# EUROPÄISCHE PATENTSCHRIFT

(11) **EP 0 994 691 B1**
(45) Veröffentlichungstag und Bekanntmachung des Hinweises auf die Patenterteilung: **24.07.2002**
(21) Anmeldenummer: 98924268.0
(22) Anmeldetag: 02.05.1998
(51) Int. Cl.: A61K 7/13

(54) **VERWENDUNG VON ESTERQUATS ALS EMULGATOREN IN ZUBEREITUNGEN FÜR DIE FÄRBUNG VON KERATINFASERN**
USE OF ESTER QUATERNARIES AS EMULSIFIERS IN PREPARATIONS FOR COLORING KERATIN FIBERS
UTILISATION D'ESTERS QUATERNAIRES DANS LES PRODUITS DE COLORATION DES CHEVEUX

(30) Priorität: 04.09.1997 GB 9718821
(43) Veröffentlichungstag der Anmeldung: 26.04.2000
(73) Patentinhaber: Cognis Deutschland GmbH & Co. KG, 40589 Düsseldorf (DE); Warner-Jenkinson Europe Ltd, Kings Lynn, Norfolk PE30 4LA (GB)
(72) Erfinder: LAWRENCE, Jacqueline, Sideup, Kent DA15 7PB (GB); MCLOUGHLIN, Francis, Wisbech, Cambridgeshire CE13 2QL (GB); PENGILLY, Roger, Kings Lynn, Norfolk PE30 4YJ (GB)
(86) Internationale Anmeldenummer: EP9802597
(87) Internationale Veröffentlichungsnummer: WO9911227

(56) Entgegenhaltungen:
- WO-A-94/21592
- GB-A- 2 168 082
- DATABASE WPI Section Ch, Week 9336 Derwent Publications Ltd., London, GB; Class D21, AN 93-285345 XP002900108 & JP 05 201835 A (KANEBO LTD) , 10 August 1993
- DATABASE WPI Section Ch, Week 8329 Derwent Publications Ltd., London, GB; Class E16, AN 83-715232 XP002900109 & SU 956 668 A (LATV LIGHT IND RES) , 7 September 1982

## Beschreibung

### Gebiet der Erfindung

Die Erfindung betrifft die Verwendung von Esterquats als Emulgator für Haarfarbstoffe in Zubereitungen für die Färbung von Keratinfasern.

### Stand der Technik

Haarfärbemittel auf synthetischer Basis können in drei Gruppen eingeteilt werden: in die direktziehenden temporären, die semipermanenten und die permanenten Färbemittel. Eine Zuordnung natürlicher Haarfärbemittel zu einer der drei Gruppen ist indes nicht ohne weiteres möglich. Ob mit einzelnen Farbstoffen eine temporäre, semipermanente oder permanente Haarfärbung zu erzielen ist, hängt u.a. auch von der Formulierung ab, in die sie eingearbeitet werden bzw. davon, wie diese Formulierungen appliziert werden.

Die mit temporären, direktziehenden Haarfärbemitteln erreichbare Färbung stellt nur eine vorübergehende Veränderung der vorhandenen Haarfarbe dar. Sie muß deshalb leicht mit Shampoos auszuwaschen sein. Sie wird durch die einfache Ablagerung der Farbstoffe auf der Oberfläche des Haares erzielt; dieser Färbung darf daher kein Ausspülvorgang folgen. Die verwendeten Farbstoffe müssen daher folgende Eigenschaften besitzen: schwache Affinität zum Haarkeratin, leichte Auswaschbarkeit durch ein Shampoo, ausreichende Lichtechtheit und gute Abriebbeständigkeit, um nicht Kleider und Kopfkissen zu verschmutzen. Für diesen Zweck kommen in der Regel Azo-, Triphenylmethan-, Anthrachinon- oder Indaminfarbstoffe sowie deren Leuko-Derivate in Frage.

Die semipermanenten Haarfärbemittel geben dem Haar stärker ausgeprägte und dauerhafte Nuancen, die bis zu 5 oder 6 Haarwäschen überstehen. Dies wird durch den Einsatz von Farbstoffen erreicht, die eine hohe Affinität zum Keratin verfügen und relativ tief in das Innere der Haarfasern eindringen. Die Aufgabe dieser Färbemittel sind vielfältig: beispielsweise einer Naturfarbe Reflexe hinzuzufügen, graue Haare abzudecken bzw. graue oder weiße Haare vom Gelbstich zu befreien. Die für diesen Zweck geeigneten direktziehenden Farbstoffe sind zum großen Teil nichtionischer oder kationischer Natur und besitzen eine niedrige relative Molmasse sowie geringe Wasserlöslichkeit. Wichtige Vertreter sind die Nitrophenyldiamine, aus denen sich durch Substitutionsreaktionen zahlreiche weitere Haarfarbstoffe herstellen lassen. Zu den Nitrofarbstoffen gehören auch die Nitroaminophenole, die - je nach Stellung ihrer Nitro- und Aminogruppe am Phenolring - Färbungen ermöglichen, die von Gelb über Orange nach Rot übergehen. Azo- oder Chinoniminfarbstoffe mit quartären Ammoniumgruppen besitzen eine hohe Affinität zum Haarkeratin. Sie färben zwar weniger intensiv als die Nitrofarbstoffe, ermöglichen jedoch Färbungen in allen Nuancen.

Die permanente Haarfärbung ist schließlich sehr beständig gegenüber Haarwäsche, Lichteinwirkung und anderen Haarbehandlungsmethoden. Hierbei werden die Farbstoffe direkt auf und im Haar gebildet, und zwar durch chemische Reaktionen, denen die ungefärbten Zwischenprodukte bzw. Vorprodukte unterworfen werden. Es laufen dabei Oxidationsreaktionen und Kupplungsvorgänge bzw. Kondensationen ab, die durch Wasserstoffperoxid in Gegenwart von Ammoniak oder Monoethanolamin hervorgerufen werden. Die Verwendung von Wasserstoffperoxid als Oxidationsmittel ist bevorzugt, da es nicht nur die Farbbildung initiiert, sondern gleichzeitig auch die Melaminpigmente des Haares zerstört und auf diese Weise eine Blondierung hervorruft. Neben den echten Oxidationshaarfarbstoffen kennt der Fachmann auch die selbstoxidierenden Farbstoffe, die bereits durch Luftsauerstoff oxidiert werden. Die Oxidationsfarbstoffe werden ihrer Natur nach in die Oxidationsbasen (Entwickler) und in die Nuancierer (Kuppler) eingeteilt. Oxidationsbasen sind aromatische Verbindungen, die mit mindestens zwei elektronenabgebenden Gruppen, vorzugsweise Amino- und/oder Hydroxygruppen, kernsubstituiert und daher leicht oxidierbar sind. Wichtige Vertreter dieser Basen sind die isomeren ortho- bzw. para-Phenyldiamine, Aminophenole und Dihydroxybenzole. Bei den Kupplern handelt es sich in der Regel ebenfalls um aromatische Verbindungen mit Amino- und/oder Hydroxygruppen, die sich jedoch in der meta-Stellung befinden.

Die Haarfarben können in verschiedensten Formen auf dem Haar appliziert werden. Es kann sich dabei im einfachsten Fall um wäßrige oder wäßrig-alkoholische Lotionen handeln, die im Augenblick der Anwendung verdünnt und auf das Haar aufgetragen werden. Eine weitere Anbietungsform sind die Kurlotionen, die neben den Farbstoffen auch vor allem Kationpolymere enthalten und somit zusätzlich einen Pflegeeffekt bewirken. Ähnliches gilt für Schaumaerosole. Eine typische Anbietungsform für die semipermanente Färbung stellen Tönungsshampoos dar, während Permanenthaarfarben in der Regel als Cremes oder Gele aufgetragen werden. Probleme insbesondere bei der Herstellung von Shampooformulierungen sowie Cremes und Gelen bestehen indes darin, einerseits den Farbstoff zusammen mit pflegenden Zusätzen stabil zu emulgieren und andererseits die rasche Ablagerung auf den Keratinfasern sicherzustellen.

Aus der russischen Patentanmeldung **SU 956.668** ist in diesem Zusammenhang ein Verfahren zur Färbung von Baumwolle bekannt, bei dem man Esterquats auf Basis von Tallölfettsäure einsetzt. Die Patentanmeldung **GB 2 168 082 A2** offenbart ein Haarfärbemittel, in dem mit kationischen Tensiden die Farbintensität auf dem Haar gesteigert wird. Ein Verfahren zur Herstellung fester Esterquats mit verbessertem Emulgiervermögen, die zur Herstellung in kosmetischen Mitteln Verwendung finden, wird in der internationalen Patentanmeldung **WO 94 21592 A** beschrieben.

Die komplexe Aufgabe der vorliegenden Erfindung hat somit darin bestanden, Emulgatoren für Haarfarbstoffe zur Verfügung zu stellen, mit deren Hilfe sich Emulsionen herstellen lassen, die auch bei längerer Lagerung oder unter Wärmeeinfluß weder separieren noch ihre Viskosität verändern. Die damit erhältlichen Zubereitungen sollten ferner zu einer verbesserten Farbaufnahme auf dem Haar führen und dieses gleichzeitig auch noch pflegen, d.h. insbesondere Griff und Glanz sowie die Kämmbarkeit verbessern.

### Beschreibung der Erfindung

Gegenstand der Erfindung ist die Verwendung von Esterquats als Emulgator für Haarfarbstoffe in Zubereitungen für die Färbung von Keratinfasern.

Überraschenderweise wurde gefunden, daß Esterquats nicht nur in der Lage sind, Haarfarbstoffe dauerhaft zu emulgieren, ohne daß die Zubereitungen ihre Viskosität verändern, mit dem Einsatz dieser Kationtenside als Emulgatoren ist auch eine erleichterte Aufnahme des Farbstoffs durch die Keratinfasern verbunden. Ein weiterer Vorteil liegt darin, daß während des Färbens den Haaren ein angenehmer Weichgriff und Glanz verliehen und die statische Aufladung zwischen den Fasern herabgesetzt wird. In diesem Sinne wird mit der erfindungsgemäßen Verwendung nicht nur die gewünschte Färbung, sondern gleichzeitig auch ein Pflegeeffekt erzielt.

### Esterquats

Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Übersichten zu diesem Thema sind beispielsweise von R.Puchta et al. in **Tens. Surf. Det., 30, 186 (1993),** M.Brock in Tens. **Surf. Det. 30, 394 (1993),** R.Lagerman et al. in **J. Am. Oil. Chem. Soc., 71, 97 (1994)** sowie I.Shapiro in **Cosm.Toil. 109, 77 (1994)** erschienen.

Die quaternierten Fettsäuretriethanolaminestersalze folgen der Formel (I), 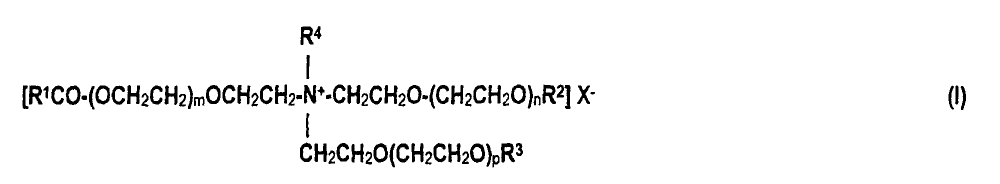 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quatemierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch quatemierte Estersalze von Fettsäuren mit Diethanolalkylaminen der Formel **(II)** in Betracht, 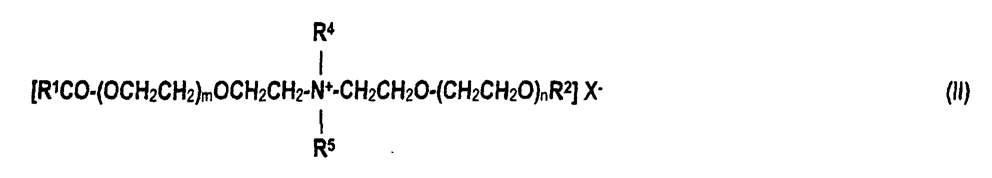 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen,

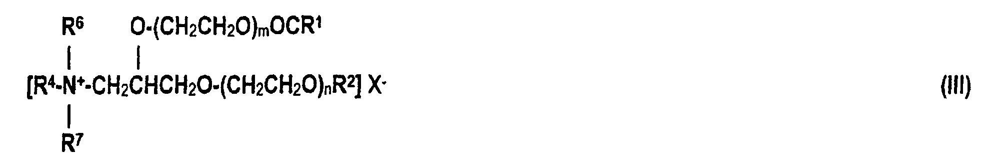

in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können. Es ist jedoch ebenfalls möglich Mischungen von Esterquats mit Fettalkoholen einzusetzen, die in Form von Schuppen im Handel erhältlich sind, und deren Herstellung im Deutschen Patent **DE-C1 4308794** (Henkel) beschrieben wird. Die Esterquats können in Mengen von 0,1 bis 5, vorzugsweise 1 bis 3 Gew.-% - berechnet auf die Zubereitungen - eingesetzt werden.

### Haarfarbstoffe

Im Sinne der erfindundungsgemäßen Verwendung kommen als Farbstoffe beispielsweise **direktziehende Farbstoffe** in Betracht, z.B. aus der Gruppe der Nitrophenylendiamine, Nitroaminophenole, Anthrachinone oder Indophenole in Betracht, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'-carbonsäure, 6-Nitro-1,2,3,4-tetrahydrochinoxalin, (N-2,3-Dihydroxypropyl-2-nitro-4-trifluormethyl)amino-benzol und 4-N-Ethyl-1,4-bis(2'hydroxyethylami-no)-2-nitrobenzol-hydrochlorid. Weiterhin auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel eingesetzt werden.

Neben den Direktziehern können auch **Oxidationsfarbstoffe**, bestehend aus Entwickler- und Kupplerkomponente verwendet werden. Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind u.a. p-Toluylendiamin, p-Aminophenol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5 und 4-Amino-3-methylphenol, 2-(2-Hydroxyethyl)-1,4-aminobenzol und 2,4,5,6-Tetraaminopyrimidin. Als Kupplerkomponenten werden in der Regel m-Phenylendiaminderivate, Naphthole, Resorcin und Resorcinderivate, Pyrazolone, m-Aminophenole sowie Pyridin-Derivate eingesetzt. Als Kupplersubstanzen eignen sich insbesondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 2-Chlor-6-methyl-3-aminophenol, 2-Methylresorcin, 2,5-Dimethylresorcin, 2,6-Dihydroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen. Die Haarfarbstoffe können in Mengen von 0,001 bis 1, vorzugsweise 0,3 bis 0,7 Gew.-% - berechnet auf die Zubereitungen - eingesetzt werden.

### Weitere Hilfs- und Zusatzstoffe

In einer besonders einfachen Ausführungsform der Erfindung enthalten die Zubereitungen zur Färbung von Keratinfasern neben den Haarfarbstoffen, den Esterquat-Emulgatoren und Wasser nur noch 01-körper. Es ist aber auch möglich Zubereitungen beispielsweise in Form von Tönungs- oder Färbeshampoos anzubieten, die dann auch weitere für die Art von Zubereitungen typische Inhaltsstoffe wie beispielsweise milde Tenside, Co-Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Parfümöle, Farbstoffe und dergleichen enthalten können.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht. Typischerweise liegt die Menge an Ölkörpern in den Zubereitungen bei 10 bis 70, vorzugsweise 25 bis 50 Gew.-%.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest gly-cosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen; sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed Collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyidiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Beispiel** 1. Durch Vermischen bei 70°C wurde eine Emulsion hergestellt, enthaltend 33,3 g Dehyquart® AU-46 (methylquaternierter Dipalmfettsäuretriethanolaminester, Methylsulfatsalz, Pulcra S.A., Barcelona), 9 g C_{8/18}-Alkylglucosid, 9 g kolloidale Kieselsäure, 3 g Ammoniumchlorid, wäßrige Ammoniaklösung ad 100 g (pH = 10,5). In die Emulsion wurde bei 20°C jeweils 7,5 mmol einer Entwicklerkomponente (N,N'-Bis(4-aminophenyl)-piperidin) und einer Kupplerkomponente (Resorcin) eingerührt. Die oxidative Entwicklung wurde anschließend mit Wasserstoffperoxid durchgeführt. Die Nuance des mit dem gefärbten Haares war dunkelblond.

**Beispiel 2.** Analog Beispiel 1 wurde eine Emulsion hergestellt, enthaltend 33,3 g Dehyquart® AU-46, 9 g C_{8/18}-Alkylglucosid, 9 g kolloidale Kieselsäure, 3 g Ammoniumchlorid, wäßrige Ammoniaklösung ad 100 g (pH = 10,5). In die PIT-Emulsion wurde bei 20°C jeweils 7,5 mmol 2,4,5,6-Tetraaminopyridin und 2,6-Bis(2-hydroxyethylamino)-toluol eingerührt. Die oxidative Entwicklung wurde wiederum mit Wasserstoffperoxid durchgeführt. Die Nuance des gefärbten Haares war tiefrot.

**Beispiele 3 bis 15.** Auf Basis dieser Beispiele wurden dann folgende Haarfärbecremeemulsion kalt hergestellt (Wasser ad 100 Gew.-%):

| | |
|---|---|
| Cremebasis nach Beispiel 1 bzw. 2 | 50,0 Gew.-% |
| Entwicklerkomponente | 7,5 mmol |
| Kupplerkomponente | 7,5 mmol |
| Na₂SO₃ (Inhibitor) | 1,0 Gew.-% |
| (NH₄)₂SO₄ | 1,0 Gew.-% |
| konz. Ammoniaklösung | ad pH 10 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt. Die oxidative Entwicklung der Färbung wurde mit Wasserstoffperoxidlösung als Oxidationslösung durchgeführt. Hierzu wurden 100 g der Emulsion mit 50 g Wasserstoffperoxidlösung (1, 3 oder 9 %ig) versetzt und vermischt. Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet. Es wurden Ausfärbungen gemäß Tabelle 1 gefunden:

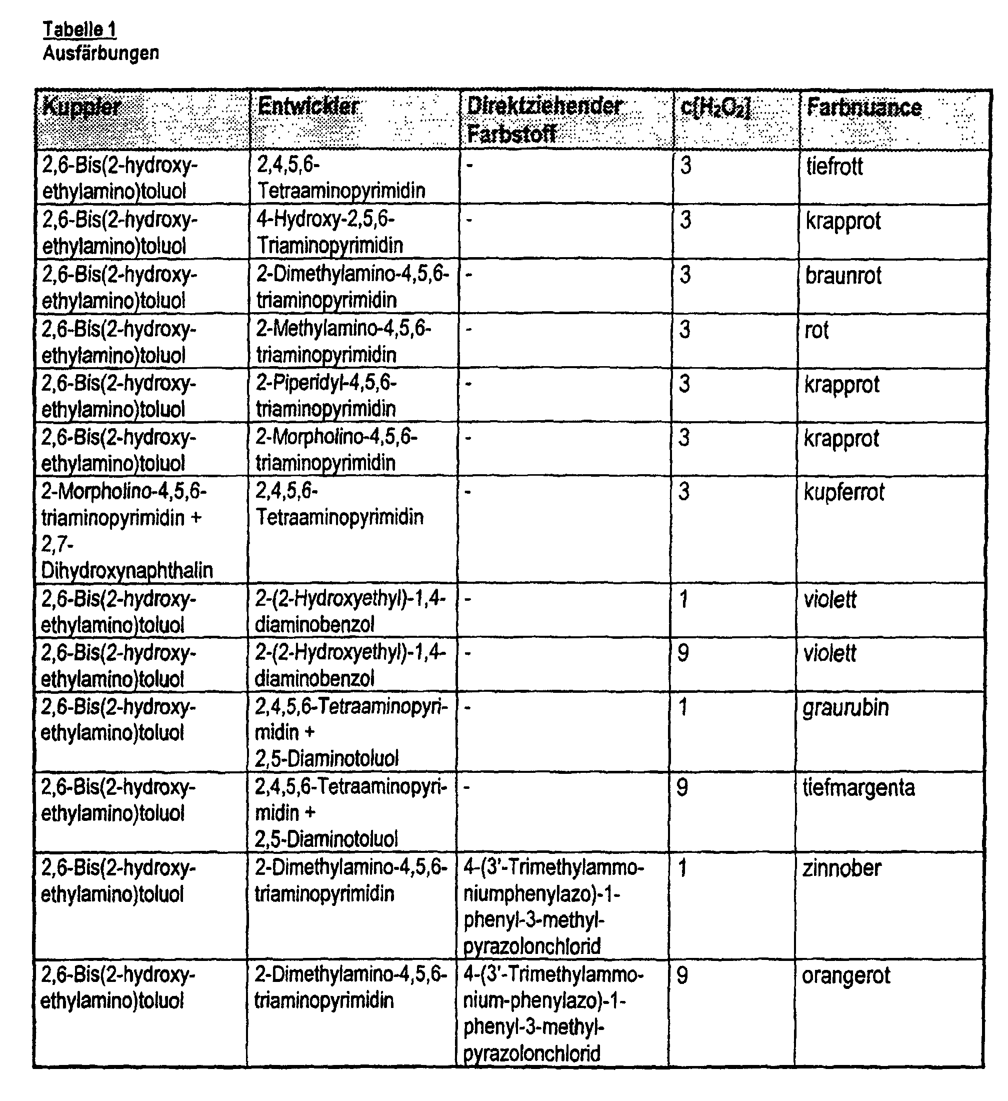

## Patentansprüche

1. Verwendung von Esterquats als Emulgator für Haarfarbstoffe in Zubereitungen für die Färbung von Keratinfasern.

2. Verwendung nach Anspruch 1, **dadurch gekennzeichnet, daß** man Esterquats der Formel **(I)** einsetzt, 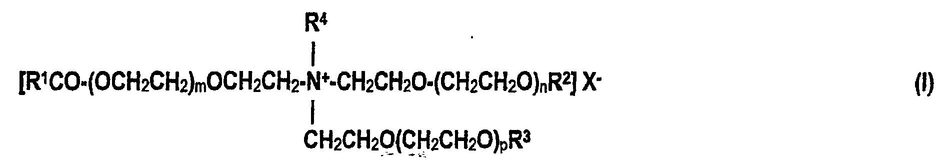 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht

3. Verwendung nach Anspruch 1, **dadurch gekennzeichnet, daß** man Esterquats der Formel **(II)** einsetzt, 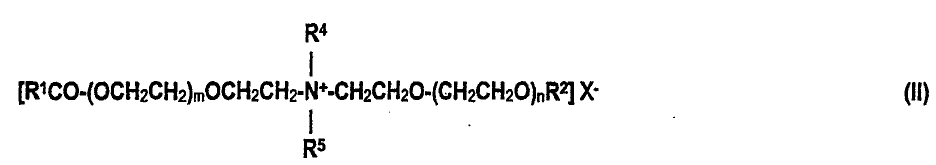 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung nach Anspruch 1, **dadurch gekennzeichnet, daß** man Esterquats der Formel **(III)** einsetzt, 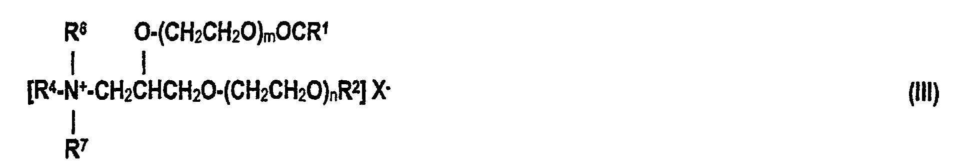 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach den Ansprüchen 1 bis 4, **dadurch gekennzeichnet, daß** man die Esterquats in Mengen von 0,1 bis 5 Gew.-% - berechnet auf die Zubreitungen - einsetzt.

6. Verwendung nach den Ansprüchen 1 bis 5, **dadurch gekennzeichnet, daß** man direktziehende Haarfarbstoffe oder Oxidationshaarfarbstoffe einsetzt.

7. Verwendung nach den Ansprüchen 1 bis 6, **dadurch gekennzeichnet, daß** man die Haarfarbstoffe in Mengen von 0,001 bis 1 Gew.-% - berechnet auf die Zubereitungen - einsetzt.

8. Verwendung nach den Ansprüchen 1 bis 7, **dadurch gekennzeichnet, daß** man weiterhin Ölkörper einsetzt, die ausgewählt sind aus der Gruppe, die gebildet wird von Guerbetalkoholen auf Basis von Fettalkoholen mit 6 bis 18 Kohlenstoffatomen, Estem von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estem von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethem, Ringöffnungsprodukten von epoxidierten Fettsäureestern mit Polyolen, Siliconölen und/oder aliphatischen bzw. naphthenischen Kohlenwasserstoffen.

9. Verwendung nach Anspruch 7, **dadurch gekennzeichnet, daß** man die Ölkörper in Mengen von 10 bis 70 Gew.-% - berechnet auf die Zubereitungen - einsetzt.

## Claims

1. The use of esterquats as emulsifiers for hair dyes in preparations for coloring keratin fibers.

2. The use claimed in claim 1, **characterized in that** esterquats corresponding to formula **(I)**: 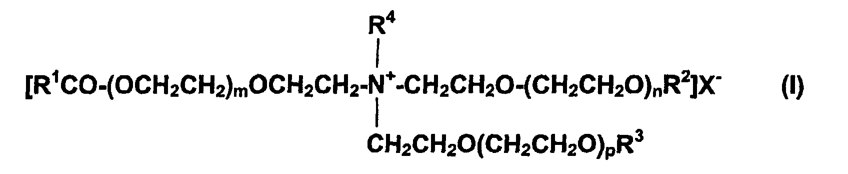 in which R¹CO is an acyl group containing 6 to 22 carbon atoms R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

3. The use claimed in claim 1, **characterized in that** esterquats corresponding to formula **(II)**: 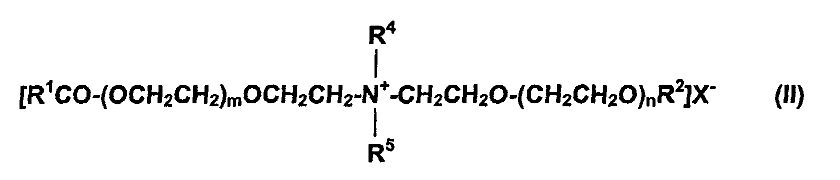 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate, are used.

4. The use claimed in claim 1, **characterized in that** esterquats corresponding to formula **(III):** 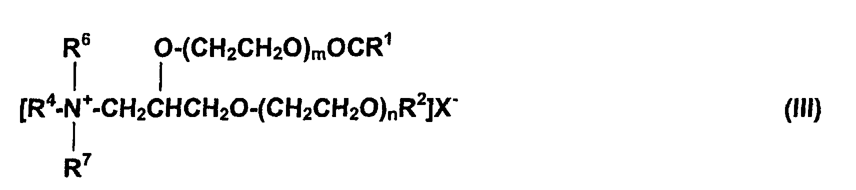 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate, are used.

5. The use claimed in claims 1 to 4, **characterized in that** the esterquats are used in quantities of 0.1 to 5% by weight, based on the preparations.

6. The use claimed in claims 1 to 5, **characterized in that** substantive hair dyes or oxidation hair dyes are used.

7. The use claimed in claims 1 to 6, **characterized in that** the hair dyes are used in quantities of 0.001 to 1% by weight, based on the preparation.

8. The use claimed in claims 1 to 7, **characterized in that** oil components selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring-opening products of epoxidized fatty acid esters with polyols, silicone oils and/or aliphatic or naphthenic hydrocarbons are additionally used.

9. The use claimed in claim 7, **characterized in that** the oils are used in quantities of 10 to 70% by weight, based on the preparations.

## Revendications

1. Utilisation d'esterquats comme agent émulsionnant pour teintures capillaires dans des préparations pour la teinture de fibres de kératine.

2. Utilisation selon la revendication 1,
**caractérisée en ce qu'**
on met en oeuvre des esterquats de formule (I) 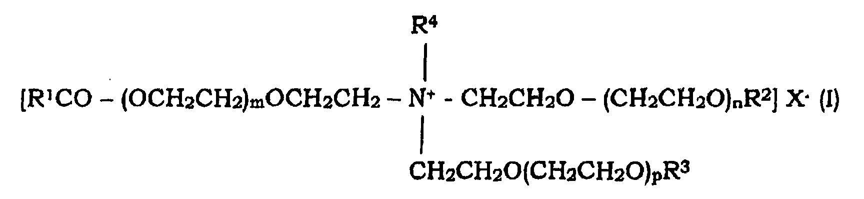 dans laquelle R¹CO représente un reste acyle ayant de 6 à 22 atomes de carbone, R² et R³ indépendamment l'un de l'autre représentent de l'hydrogène ou R¹CO, R⁴ représente un reste alkyle ayant de 1 à 4 atomes de carbone ou un groupe (CH₂CH₂O)_{q}H, m, n et p globalement représentent 0 ou des nombre allant de 1 à 12, q représente des nombres allant de 1 à 12 et X représente un halogénure, un alkylsulfate ou un alkylphosphate.

3. Utilisation selon la revendication 1,
**caractérisée en ce qu'**
on met en oeuvre des esterquats de formule (II) 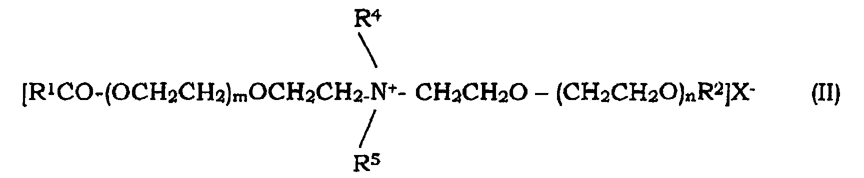 dans laquelle R¹CO représente un reste acyle ayant de 6 à 22 atomes de carbone, R² représente de l'hydrogène ou R¹CO, R⁴ et R⁵, indépendamment l'un de l'autre, représentent des restes alkyle ayant de 1 à 4 atomes de carbone, m et n globalement représentent 0 ou des nombres allant de 1 à 12 et X représente un halogénure, un alkylsulfate ou un alkylphosphate.

4. Utilisation selon la revendication 1,
**caractérisée en ce qu'**
on met en oeuvre des esterquats de formule générale III 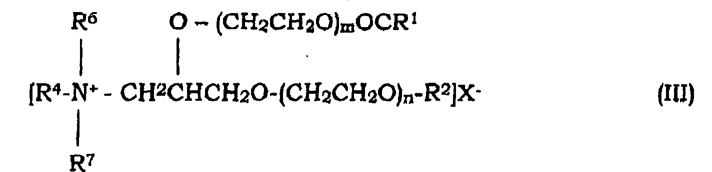 dans laquelle R¹CO représente un reste acyle ayant de 6 à 22 atomes de carbone, R² représente de l'hydrogène ou R¹CO, R⁴, R⁶ et R⁷, indépendamment les uns des autres, représentent des restes alkyle ayant de 1 à 4 atomes de carbone, m et n globalement représentent 0 ou des nombres allant de 1 à 12, et X représente un halogène, un alkylsulfate ou un alkylphosphate.

5. Utilisation selon les revendications 1 à 4,
**caractérisée en ce qu'**
on met en oeuvre les esterquats en quantité allant de 0,1 à 5% en poids calculé sur les préparations.

6. Utilisation selon les revendications 1 à 5,
**caractérisée en ce qu'**
on met en oeuvre des teintures capillaires à action directe ou par oxydation.

7. Utilisation selon les revendications 1 à 6,
**caractérisée en ce qu'**
on met en oeuvre les teintures capillaires en quantité allant de 0,001 à 1% en poids, calculé sur les préparations,

8. Utilisation selon les revendications 1 à 7,
**caractérisée en ce qu'**
on met en oeuvre en outre des composés huileux choisis dans le groupe formé par des alcools de Guerbet à base d'alcools gras ayant de 6 à 18 atomes de carbone, des esters d'acide gras linéaire en C₆-C₂₂ avec des alcools gras linéaires en C₆-C₂₂, des esters d'acides carboxyliques ramifiés en C₆-C₁₃ avec des alcools gras linéaires en C₆-C₂₂, des esters d'acides gras linéaires en C₆-C₂₂ avec des alcools ramifiés, des esters d'acides gras linéaires et/ou ramifiés avec des alcools plurifonctionnels et/ou des alcools de Guerbet, des triglycérides à base d'acides gras en C₆-C₁₀, des mélanges liquides de mono-/de di- et de triglycérides à base d'acides gras en C₆-C₁₈, des esters d'alcools gras en C₆-C₂₂ et/ou d'alcools de Guerbet avec des acides carboxyliques aromatiques, des huiles végétales, des alcools primaires ramifiés, des cyclohexanes substitués, des carbonates d'alcools gras en C₆-C₂₂, des carbonates de Guerbet, des esters d'acide benzoïque avec des alcools linéaires et/ou ramifiés en C₆-C₂₂, des esters dialkyliques, des produits d'ouverture du cycle d'esters d'acide gras époxydé avec des polyols, des huiles de silicone, et/ou des hydrocarbures aliphatiques ou naphténiques.

9. Utilisation selon la revendication 7,
**caractérisée en ce qu'**
on met en oeuvre les composés huileux en quantité allant de 10 à 70% en poids calculé sur les préparations.
